# EUROPÄISCHE PATENTSCHRIFT

(11) **EP 0 574 800 B1**
(45) Veröffentlichungstag und Bekanntmachung des Hinweises auf die Patenterteilung: **21.08.1996**
(21) Anmeldenummer: 93109174.8
(22) Anmeldetag: 08.06.1993
(51) Int. Cl.: C14C 3/08, C07D 309/10

(54) **Verfahren zum Gerben von Leder und Pelzen**
Process for tanning leather and furs
Procédé de tannage du cuir et fourrures

(30) Priorität: 13.06.1992 DE 4219419
(43) Veröffentlichungstag der Anmeldung: 22.12.1993
(73) Patentinhaber: BASF Aktiengesellschaft, 67063 Ludwigshafen (DE)
(72) Erfinder: Greif, Norbert, Dr., D-6719 Bobenheim (DE); Oppenlaender, Knut, Dr., D-6700 Ludwigshafen (DE); Birkhofer, Hermann, Dr., W-6700 Ludwigshafen (DE); Wegner, Brigitte, Dr., D-6725 Roemerberg (DE); Dix, Johannes Peter, Dr., D-6719 Weisenheim (DE)

(56) Entgegenhaltungen:
- FR-A- 2 237 963

## Beschreibung

Die Erfindung betrifft ein verbessertes Verfahren zum Alleingerben, Vorgerben und Mitgerben von Blößen und Fellblößen und zum Nachgerben von Leder und Fell.

Aldehyde und insbesondere Dialdehyde wie Glutardialdehyd werden als Ledergerbstoffe oft in der Literatur beschrieben und häufig in der Praxis eingesetzt. So betrifft beispielsweise die DE-A-17 69 059 ein Verfahren zur Lederherstellung durch Behandeln von gerbbarem Material mit Zinksalzen, wasserlöslichen Sulfaten und Aldehyden oder Aldehyde abspaltenden Stoffen und Nachbehandeln mit Abstumpfungsmitteln.

Diese Verfahren und die durch diese Verfahren erhaltenen Leder und Felle weisen aber häufig eine Reihe von Nachteilen auf. Bei der Gerbung insbesondere mit Glutardialdehyd können aufgrund seiner hohen Reaktivität ungleichmäßige Gerbeffekte auftreten; dies ist insbesondere bei ungespaltenen Blößen der Fall, bei denen mit niedrigen Konzentrationen von Dialdehyd keine vollständige Durchgerbung erzielt wird. Weiterhin gestalten zu hohe Flüchtigkeiten und oft zu hohe Toxizitäten oder Gesundheitsgefährdungspotentiale der verwendeten Aldehyde die Gerbeverfahren problematisch, weil hierdurch Schutzmaßnahmen getroffen werden müssen und außerdem eine relativ hohe Einsatzmenge an Gerbstoff notwendig ist.

Aufgabe der vorliegenden Erfindung war es daher, neue Gerbstoffe für die Allein-, Vor-, Mit- und Nachgerbung bereitzustellen, die die Nachteile der Mittel des Standes der Technik nicht mehr aufweisen.

Die Erfindung betrifft nun ein Verfahren zum Gerben von Leder, das dadurch gekennzeichnet ist, daß man dazu Verbindungen der allgemeinen Formel I 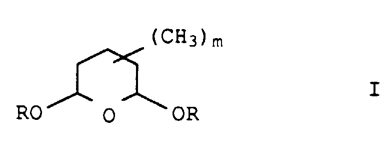 verwendet, in der
- m: die Zahlen 0 oder 1 und die Reste
- R: unabhängig voneinander gegebenenfalls durch Hydroxy- oder Carboxy- substituiertes und/oder durch Sauerstoffatome unterbrochenes C₁- bis C₁₈-Alkyl oder einer der Reste R auch einen Rest der Formel 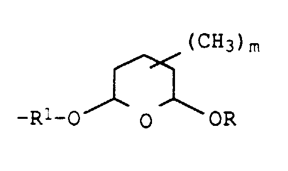 bedeuten, wobei
- R¹: gegebenenfalls durch Hydroxy substituiertes und/ oder durch Sauerstoffatome unterbrochenes Alkylen ist.

C₁- bis C₁₈-Alkylreste R können linear oder verzweigt sein, wobei die C₉- bis C₁₈-Reste, z.B. Nonyl, Decyl, Dodecyl, Tridecyl, Tetradecyl, Hexadecyl oder Octadecyl weniger bevorzugt sind.

Hydroxycarbonsäurereste R stammen z.B. von folgenden Säuren: Hydroxyessigsäure, Hydroxypropionsäure, Milchsäure oder Hydroxystearinsäure.

Die Reste R können dabei natürlich auch in Salzform vorliegen, z.B. als Lithium-, Natrium-, Kalium- oder gegebenenfalls substituierte Ammoniumsalze.

Reste R sind insbesondere C₁- bis C₈-Alkyl sowie C₃- bis C₄₀-Oxaalkyl wie CH₃, C₂H₅, C₃H₇, C₄H₉, C₅H₁₁, C₆H₁₃, C₇H₁₅, C₈H₁₇, 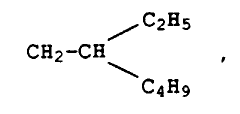 CH₂CH₂OCH_{3,} CH₂CH₂OC₂H₅, CH₂CH₂-OC₄H₉, CH₂CHOH-CH₂ (CH₂CH₂O)₂-CH₃, (CH₂CH₂O)₃CH₃, (CH₂CH₂O)₄CH₃ oder Polyalkylenoxidgruppen.

Eine bevorzugte Gruppe von Resten R entspricht der Formel 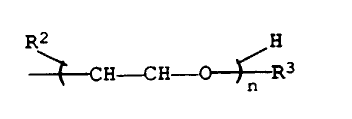 in der
- n: 1 bis 20,
- R²: Wasserstoff oder C₁- bis C₄-Alkyl und
- R³: Wasserstoff oder C₁- bis C₈-Alkyl sind.

Reste R¹ entsprechen insbesondere der Formel 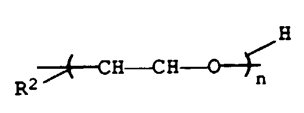 in der R² und n die angegebenen Bedeutungen haben.

Aufgrund der Herstellung der Verbindungen der Formel I ist einer der Reste R vorzugsweise C₁- bis C₄-Alkyl, insbesondere Methyl.

Bevorzugte Verbindungen entsprechen der Formel Ia 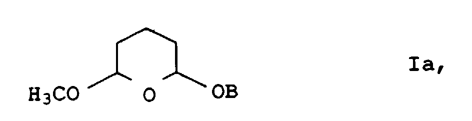 wobei
B ein Rest der Formel 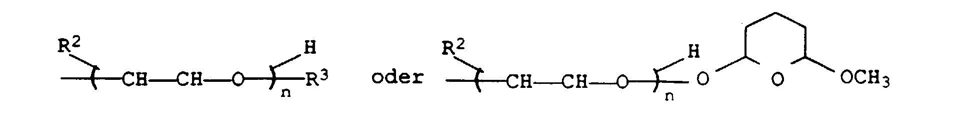 ist, und n, R² und R³ die angegebenen Bedeutungen haben.

R² ist vorzugsweise Wasserstoff und n 4 bis 10.

Erfindungsgemäß ist es wichtig, daß die Verbindungen der Formel I möglichst gut wasserlöslich sind. Durch Variation und Kombination der verschiedenen Substituenten ist es dem Fachmann leicht möglich, diese Wasserlöslichkeit zu erzielen.

Die erfindungsgemäß zu verwendenden Verbindungen der Formel 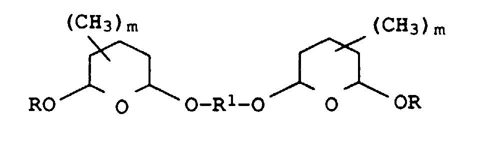 sind neu; hinsichtlich der Bevorzugungen gilt das bereits Gesagte.

Zur Herstellung der Verbindungen der Formel I kann man in an sich bekannter Weise alkoholische Hydroxylverbindungen an 2-Alkoxy-3,4-dihydro-2-pyrane anlagern; bevorzugt als Pyranverbindung ist 2-Methoxy-3,4-dihydro-2-pyran. Einzelheiten der Herstellung können den entsprechenden Beispielen entnommen werden.

Das erfindungsgemäße Gerbverfahren eignet sich in hervorragender Weise zum Alleingerben und Vorgerben von Blößen und Fellblößen in wäßriger Flotte. Hierbei geht man zweckmäßigerweise so vor, daß die gepickelten Blößen, beispielsweise Rindsblößen mit einer Spaltstärke von 1,5 bis 4 mm, oder Fellblößen, beispielsweise Schaffellblößen, bei einem pH-Wert von 2 bis 7, insbesondere 2,5 bis 4, und einer Temperatur von 15 bis 50°, insbesondere 25 bis 45°C, während eines Zeitraumes von 3 bis 20 Stunden mit einer wäßrigen Lösung oder Dispersion der Verbindungen I behandelt werden. Die Behandlung erfolgt beispielsweise durch Walken in einem Faß. Die benötigte Menge an Verbindungen I beträgt normalerweise, bezogen auf das Blößengewicht, 2 bis 30 Gew.-%, insbesondere 5 bis 20 Gew.-%. Die Flottenlänge, d.h. das prozentuale Gewichtsverhältnis der Behandlungsflotte zur Ware, beträgt üblicherweise 30 bis 200 % bei Blößen und 100 bis 2000 % bei Fellblößen, jeweils bezogen auf das Blößengewicht.

Nach erfolgter Behandlung wird das Leder bzw. Fell üblicherweise auf einen pH-Wert von 4 bis 8, insbesondere 5 bis 7, eingestellt, wozu man beispielsweise Magnesiumoxid, Natriumcarbonat oder Natriumhydrogencarbonat verwendet, gegebenenfalls mit weiteren Gerbstoffen behandelt und nach Abschluß des Gerbeprozesses gewünschtenfalls gefärbt und gefettet.

Das erfindungsgemäße Gerbverfahren eignet sich ebenfalls in hervorragender Weise zum Mitgerben von Blößen und Fellblößen zusammen mit den Gerbstoffen der Hauptgerbung, welche beispielsweise eine Chrom- oder eine Aluminiumgerbung sein kann. In diesem Fall werden die Arbeitsbedingungen bezüglich pH-Wert, Temperatur und Dauer der Behandlung auf die Anforderungen der Hauptkomponenten der Gerbung eingestellt, das gleiche gilt für die Behandlungsapparatur und die Flottenlänge sowie für die Nachbehandlung. Die benötigte Menge an Verbindungen I beträgt hierbei normalerweise, bezogen auf das Blößengewicht, 0,1 bis 20 Gew.-%, insbesondere 0,5 bis 15 Gew.-%.

Das erfindungsgemäße Gerbverfahren eignet sich ebenfalls in hervorragender Weise zum Nachgerben von bereits gegerbtem Leder und Fell, beispielsweise Chromleder, in wäßriger Flotte. Es wird in der Regel so durchgeführt, daß man die gepickelten Blößen und Felle, beispielsweise Rindsblößen mit Spaltstärken von 1,5 bis 4 mm, mit beispielsweise einem üblichen chromhaltigen Gerbstoff wie einem Chrom(III)-Salz, z.B. Chrom(III)-Sulfat, in an sich bekannter Weise gerbt, die so erhaltenen vorgegerbten Häute (bei Chromgerbung "Wetblues") entsäuert und bei einem pH-Wert von 2 bis 7, insbesondere 2,5 bis 4, und bei Temperaturen von 15 bis 50°C, insbesondere 25 bis 45°C, während eines Zeitraumes von 1 bis 12 Stunden mit einer wäßrigen Lösung oder Dispersion der Verbindungen I behandelt. Diese Behandlung erfolgt beispielsweise durch Walken in einem Faß. Die benötigte Menge an Verbindungen der Formel I beträgt normalerweise, bezogen auf das Falzgewicht des Leders, 2 bis 30 Gew.-%, insbesondere 5 bis 20 Gew.-%. Die Flottenlänge beträgt üblicherweise 30 bis 200 % bei Blößen und 100 bis 2000 % bei Fellblößen, jeweils bezogen auf das Falzgewicht des Leders.

Nach und erforderlichenfalls auch vor der Behandlung wird das Leder bzw. Fell üblicherweise auf einen pH-Wert von 3 bis 5 eingestellt, wozu man beispielsweise Magnesiumoxid oder eine organische Säure wie Ameisensäure oder deren Salze verwendet, und nach der Behandlung gewünschtenfalls gefärbt und gefettet.

Das erfindungsgemäß nachgegerbte Leder oder Fell kann vor der Nachgerbung mit den Verbindungen I zusätzlich mit anderen Gerbstoffen wie Polymergerbstoffen oder Syntanen behandelt worden sein. Auch können die Verbindungen I gleichzeitig mit derartigen zusätzlichen Gerbemitteln, beispielsweise in der Hauptgerbung, eingesetzt werden.

Als zusätzliche oder gleichzeitig eingesetzte Gerbstoffe kommen alle üblichen Mittel mit Gerbwirkung auf Blößen oder Fellblößen in Betracht. Eine umfassende Abhandlung derartiger Gerbstoffe findet sich beispielsweise in Ullmanns Encyklopädie der technischen Chemie, 3. Auflage, 11. Band, Seiten 585 bis 612 (1960). Einzeln zu erwähnende Gerbstoffklassen sind die mineralischen Gerbstoffe, z.B. Chrom-, Aluminium-, Zink- und Zirkoniumsalze, die synthetischen Gerbstoffe wie die bereits oben genannten Polymergerbstoffe und Syntane, und die vegetabilischen (pflanzlichen) Gerbstoffe.

Beim erfindungsgemäßen Gerbverfahren entstehen Leder und Pelze, die im Vergleich zu den mit bisher bekannten Aldehydgerbstoffen wie Glutardialdehyd erhaltenen Erzeugnissen neben einem vollen und sehr weichen Griff und neben hohen Schrumpfungstemperaturen eine deutlich verbesserte Reiß- und Zugfestigkeit aufweisen. Zudem weisen die erfindungsgemäß gegerbten Leder und Felle keine störende gelbe Farbe mehr auf. Ein klarer Vorteil ist das gute Penetrationsvermögen bereits geringer Mengen der erfindungsgemäß zu verwendenden Verbindungen.

Ein weiterer Vorteil des erfindungsgemäßen Gerbverfahrens ist die geringe Flüchtigkeit der Verbindungen der Formel I, was sich beispielsweise auf die Einsatzmenge dieser Gerbstoffe auswirkt, welche nur verhältnismäßig gering zu sein braucht. Weiterhin stellen die Verbindungen I in gewissem Sinne Universalgerbstoffe dar, da sie mit allen anderen üblichen Gerbstoffen kombiniert werden können und sowohl für die Allein-, die Vor-, die Mit als auch für die Nachgerbung verwendbar sind.

### Allgemeine Herstellvorschrift für Verbindungen der Formel I

Die Addition OH-gruppenhaltiger Produkte an 2-Methoxy-3,4-dihydro-2-pyran erfolgt säurekatalysiert bei 50 bis 100°C. Als Säuren kommen Mineral-, Sulfon- oder Carbonsäuren in Betracht. Die Reaktion kann IR-spektroskopisch verfolgt werden, da die C = C - Doppelbindungsbande bei 1650 cm⁻¹ bei der Anlagerung verschwindet.

### Beispiel 1

Zu 288 g eines Anlagerungsproduktes von Ethylenoxid an Glykol mit dem Molgewicht von - 300 und 0,5 g p-Toluolsulfonsäure werden bei 70°C 219 g 2-Methoxy-3,4-dihydro-2-pyran (MOP) zugetropft, wobei die Temperatur auf 85° ansteigt. Das Reaktionsgemisch wird anschließend 30 min bei 85° nachgerührt. Unter vermindertem Druck werden dann noch Spuren von nicht umgesetzten MOP entfernt.
Ausbeute: 497,5 g
IR bei 1650 cm-1: geringe Absorption

### Beispiel 2

Analog beispiel 1 erhält man bei der Umsetzung von MOP mit Glycerin folgendes Resultat:
92,1 g Glycerin
1 g p-Toluolsulfonsäure
114 g MOP
Ausbeute: 205,5 g IR (1650 cm⁻¹) = gering

### Beispiel 3

Umsetzungsprodukt von MOP mit Glycolsäure
Reaktionsführung wie Beispiel 1
97,3 g Glycolsäure
146 g MOP
Ausbeute: 242,3 g IR (1650 cm⁻¹) = geringe Absorption.

### Anwendungsbeispiele

### Beispiel 4

### Alleingerbung von Rindsblößen

Gut entkälkte und gepickelte Rindsblöße mit einer Spaltstärke von 2 mm wurde bei einer Flottenlänge von 100 % in drei Portionen mit 10 Gew.-%, bezogen auf das Pickelgewicht der gemäß Beispiel 3 hergestellten Verbindung versetzt und 3 Stunden bei Raumtemperatur und einem pH-Wert von ca. 3 im Faß gewalkt. Anschließend wurde auf 40°C erwärmt und mit Magnesiumoxid ein pH-Wert von ca. 7 eingestellt. Nach kurzem Spülen wurde das Leder wie üblich fertiggestellt.

Man erhielt ein weißes Leder mit einer Schrumpfungstemperatur von ca. 85°C, welches sehr weich und griffig war.

### Beispiel 5

Analog Beispiel 4 wurden 8 Gew.-% Gerbstoff, hergestellt gemäß Beispiel 1 zur Alleingerbung eingesetzt. Das damit erhaltene Leder besaß eine Schrumpfungstemperatur von ca. 83°C.

### Beispiel 6

Verwendung der Verbindungen der Formel I in der Vorgerbung:

Gut entkälkte und gepickelte Rindsblöße mit einer Spaltstärke von 2 mm wurde bei einer Flottenlänge von 50 % zunächst mit 1 % Fettlicker 10 Min., dann nach Zusatz von ca. 0,7 % Gerbstoff, hergestellt gemäß Beispiel 1 und 1 % Polymergerbstoff nochmals 60 Min. im Faß gewalkt. Anschließend wurde 1 % eines synthetischen Gerbstoffes zugegeben und nochmals 60 Min. im Faß gewalkt. Der pH-Wert wurde dann über Nacht mit Magnesiumoxid auf 3,9 eingestellt.

Man erhält so ein sehr helles, vorgegerbtes Leder, mit einer Schrumpfungstemperatur von ca. 70°C, das sich problemlos falzen läßt. Durch Ausgerben mit Chrom- bzw. Aluminiumsalzen, synthetischen oder auch vegetabilischen Gerbstoffen können aus dieser Zwischenstufe sämtliche Lederarten hergestellt werden.

### Beispiel 7

### Nachgerbung von Chromrindleder

Ein in üblicher Weise hergestelltes und auf 1,5 mm gefalztes Rindwetblue wurde in 100 % Flotte mit 2,5 Gew.-% Gerbstoff, (synthetisiert gemäß Beispiel 1) bezogen auf das Falzgewicht, versetzt und 90 Min. bei 40°C und einem pH-Wert von ca. 3,4 im Faß gewalkt. Anschließend wurde der pH-Wert mit 1 Gew.-% Natriumformiat und 0,5 Gew.-% Natriumhydrogencarbonat auf ca. 4,6 eingestellt.

Nach der darauffolgenden Färbung und Fettung mit handelsüblichen Produkten und der üblichen Fertigstellung erhielt man ein weiches, volles Leder mit angenehmem Griff.

### Beispiel 8

### Nachgerbung für weiches Rindboxleder

Auf eine Stärke von 1,5 mm gefalztes Rindwetblue wurde zunächst gewaschen und anschließend in 50 Gew.-% Flotte (Prozentangaben beziehen sich auf Falzgewicht) mit 2 Gew.-% eines handelsüblichen schwach maskierten Chromgerbstoffes und 2,5 Gew.-% analog Beispiel 1 hergestelltem Gerbstoff 45 Min. im Faß gewalkt.

Anschließend wurde der pH-Wert mit 1 Gew.-% Natriumformiat auf ca. 4 eingestellt. Nach erneutem Waschen wurde das nachgegerbte Leder mit einem üblichen Lederfarbstoff gefärbt, mit einem üblichen Licker gefettet und in üblicher Weise fertiggestellt.

Man erhielt ein sehr weiches, volles und griffiges Leder mit sehr egaler Färbung.

## Patentansprüche

1. Verfahren zum Gerben von Leder und Pelzen, dadurch gekennzeichnet, daß man dazu Verbindungen der allgemeinen Formel I 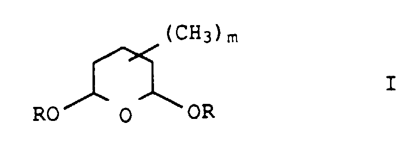 verwendet, in der
m die Zahlen 0 oder 1 und die Reste
R unabhängig voneinander gegebenenfalls durch Hydroxy- oder Carboxy- substituiertes und/oder durch Sauerstoffatome unterbrochenes C₁- bis C₁₈-Alkyl oder einer der Reste R auch einen Rest der Formel 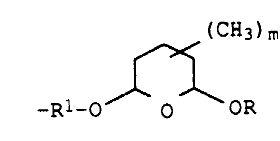 bedeuten, wobei
R¹ gegebenenfalls durch Hydroxy substituiertes und/oder durch Sauerstoffatome unterbrochenes Alkylen ist.

2. Verfahren gemäß Anspruch 1, dadurch gekennzeichnet, daß man dazu Verbindungen der Formel 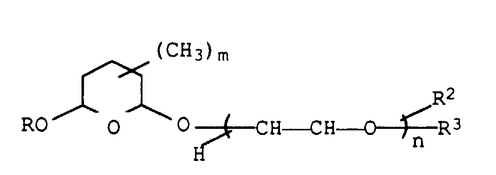 oder 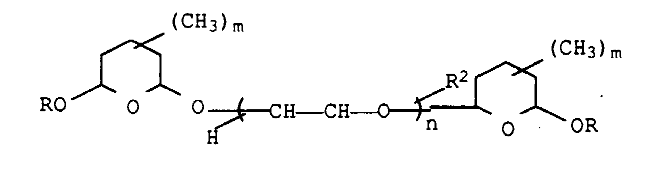 verwendet, wobei
n die Zahlen 1 bis 20,
R² Wasserstoff oder C₁- bis C₄-Alkyl und
R³ Wasserstoff oder C₁- bis C₈-Alkyl bedeuten und m und R die angegebene Bedeutung haben.

3. Verfahren gemäß Abspruch 2, dadurch gekennzeichnet, daß man dazu Verbindungen der Formel 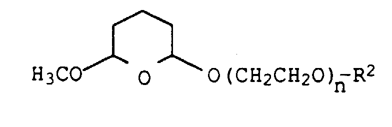 oder 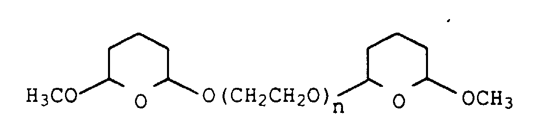 verwendet, wobei n und R² die angegebene Bedeutung haben.

4. Verbindungen der Formel 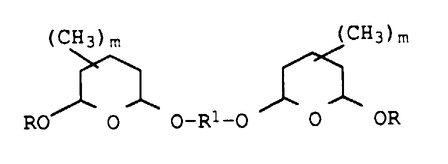 in der m, R und R¹ die für Anspruch 1 angegebene Bedeutung haben.

5. Verbindungen der Formel gemäß Anspruch 4, bei denen m = O und R Methyl sind.

## Claims

1. A process for tanning leather and fur, which comprises using for this purpose compounds of the general formula I 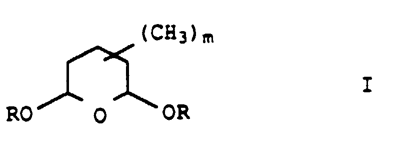 where
m is 0 or 1, and the radicals
R are independently of each other C₁-C₁₈-alkyl, which may be substituted by hydroxyl or carboxyl and/or interrupted by oxygen atoms, or else one of the radicals R is a radical of the formula 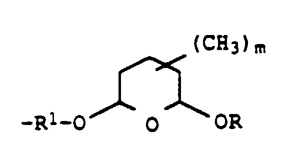 where
R¹ is alkylene, which may be substituted by hydroxyl and/or interrupted by oxygen atoms.

2. A process as claimed in claim 1, wherein the compound used has the formula 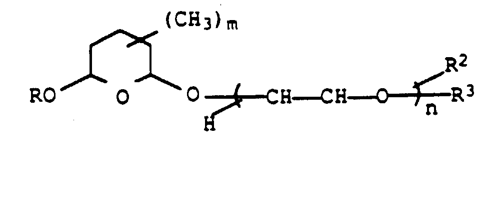 or 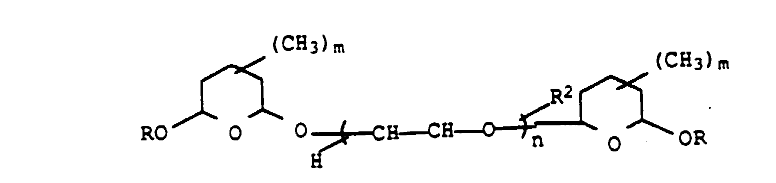 where
n is from 1 to 20,
R² is hydrogen or C₁-C₄-alkyl, and
R³ is hydrogen or C₁-C₈-alkyl, and m and R are each as defined above.

3. A process as claimed in claim 2, wherein the compounds used have the formula 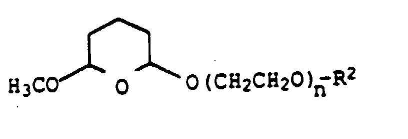 or 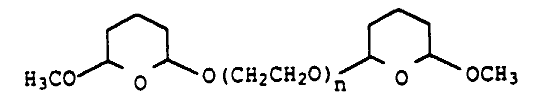 where n and R² are each as defined above.

4. Compounds of the formula 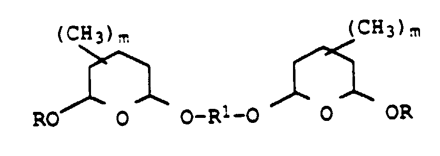 where m, R and R¹ are each as defined for claim 1.

5. Compounds of the formula as set forth in claim 4 where m is 0 and R is methyl.

## Revendications

1. Procédé de tannage du cuir et de peaux, caractérisé en ce que l'on utilise, à cette fin, des composés de la formule générale I : 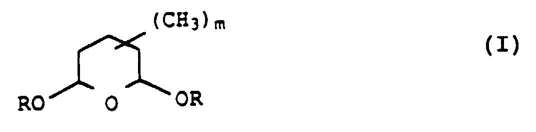 dans laquelle
m est égal à 0 ou à 1, et
les symboles R représentent, chacun indépendamment l'un de l'autre, un radical alkyle en C₁-C₁₈ éventuellement substitué par des groupes hydroxyle ou carboxyle et/ou interrompu par des atomes d'oxygène, ou bien l'un des symboles R représente aussi un reste de la formule : 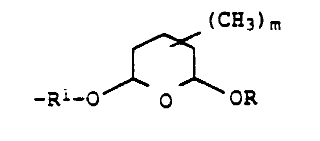 où
le symbole R¹ représente un groupe alkylène éventuellement substitué par des radicaux hydroxyle et/ou interrompu par des atomes d'oxygène.

2. Procédé selon la revendication 1, caractérisé en ce que l'on utilise, à cette fin, des composés de la formule : 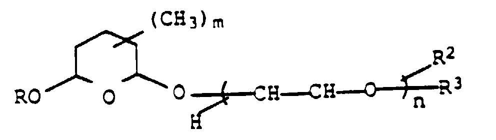 ou 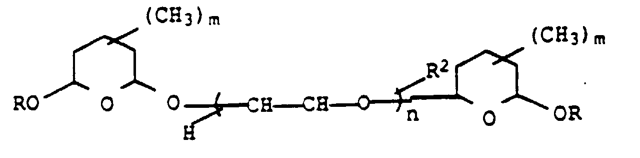 dans lesquelles
n est un nombre dont la valeur varie de 1 à 20,
R² représente un atome d'hydrogène ou un radical alkyle en C₁-C₄, et
R³ représente un atome d'hydrogène ou un radical alkyle en C₁-C₈ et m et R on les significations qui leur ont été précédemment attribuées.

3. Procédé selon la revendication 2, caractérisé en ce que l'on utilise, à cette fin, des composés des formules : 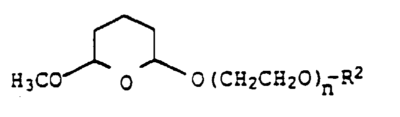 ou 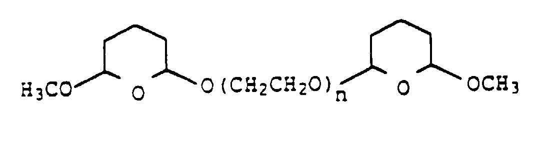 dans lesquelles
n et R² possèdent les significations qui leur ont été précédemment attribuées.

4. Composés de la formule : 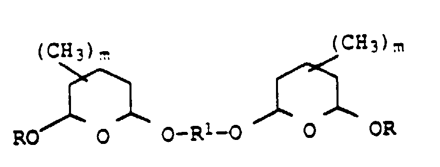 dans laquelle
m, R et R¹ ont les significations qui leur ont été attribuées dans la revendication 1.

5. Composés de la formule selon la revendication 4, dans lesquels m est égal à 0 et R représente le radical méthyle.
